# EUROPEAN PATENT APPLICATION

(11) **EP 4 692 045 A1**
(43) Date of publication of application: **11.02.2026**
(21) Application number: 24192795.3
(22) Date of filing: 05.08.2024
(51) Int. Cl.: C07C 209/62, C07C 211/42, C12N 9/20, C12P 13/00, C12P 13/02, C12P 41/00

(54) **ENZYMATIC PROCESS FOR THE SEPARATION OF 2,6-DIMETHYL-2,3-DIHYDRO-1H-INDEN-1-AMINE STEREOISOMERS**

(71) Applicant: Adama Agan Ltd., 7710201 Ashdod (IL)
(72) Inventor: WELLS, Andrew, Leicestershire UK LE12 8AT (GB); PARNES, Regev, Hanegev 101 (IL); ZOAIDA, Sami, Rahat 76, 31 (IL); BAR-NAHUM, Itsik, Gibton (IL); FINE, Sergey, Baz 4 (IL)
(74) Representative: Modiano, Gabriella Diana

(57) **Abstract**

An enzymatic process for the separation of isomers of 2,6-dimethyl-2,3-dihydro-1H-inden-1-amine.

## Description

### FIELD OF THE INVENTION

The present invention belongs is directed to a process for preparing virtually enantiomerically pure (1R,2S)-2,6-dimethyl-2,3-dihydro-1H-inden-1-amine. This compound is a useful intermediate in the synthesis of triazines, which are suitable as herbicides for controlling weeds.

### BACKGROUND

Triazine compounds are a class of compounds suitable for being used as herbicides. Triazine compounds such as atrazine, ametryne, indaziflam or triaziflam are among the compounds that are used as herbicides.

The prior art discloses in EP1592674 A1, EP2231679 A1 or EP3347342 A1 different methods for preparing indaziflam. One of the methods disclosed in the prior art document EP1592674 A1 relates to the preparation of indaziflam from the reaction of 1-cyanoguanidine with the amine compound (1R,2S)-2,6-dimethyl-2,3-dihydro-1H-inden-1-amine. In that document, enantiomerically pure 1-indanamine compounds with only 1 chiral center are proposed to be prepared by enantioselective acylation of racemic 1-indanamine compounds in the presence of a biocatalyst. The enantioselectively acylated enantiomer is cleaved back to the corresponding amine with an acid or a base. Some lipases like for example, *Pseudomonas cepacia, Candida cylindracea* or *Candida antarctica* are proposed as biocatalysts. Particularly, *Candida antarctica* is disclosed in immobilized form (commercial name Novozym 435) as biocatalyst in an example for the reaction of a mixture of trans-1-amino-2-methylindane (i.e. (1S,2R)- and (1R,2S)-1-amino-2-methylindane) and methyl 2-methoxyacetate in tert-butyl methyl ether as solvent at reflux (i.e. 55ºC).

In document WO2024/041962, a process for the separation of (1R,2S)-2,6-dimethyl-2,3-dihydro-1H-inden-1-amine or (1R,2S)-1-amino-2,6-dimethylindane has been also disclosed. The process contains 4 steps, wherein the step a) is the acylation or carboxylation step of the mixture of the 4 stereoisomers of 2,6-dimethyl-2,3-dihydro-1H-inden-1-amine to yield an amide or carbamate compound of (1R,2S)-2,6-dimethyl-2,3-dihydro-1H-inden-1-amine; step b) is the production of the 4 stereoisomers of 2,6-dimethyl-2,3-dihydro-1H-inden-1-amine from a mixture of the 3 stereoisomers (1S,2S)-, (1R,2R)-and (1S,2R)- of 2,6-dimethyl-2,3-dihydro-1H-inden-1-amine by hydrogenation with a metal catalyst; step c) is the separation by crystallization of the amide or carbamate compound of the (1R,2S)-2,6-dimethyl-2,3-dihydro-1H-inden-1-amine; and step d) is the reaction of this amide or carbamate compound with a base or an acid to produce the (1R,2S)-2,6-dimethyl-2,3-dihydro-1H-inden-1-amine. The acylation or carboxylation of step a) is catalyzed by a protein sequence of an uncultured bacterium with Lipase activity and CAS number 3029749-54-7, in a solvent selected from methyl t-butyl ether, heptane, toluene, xylenes, mesitylene, anisole, chlorobenzene, n-butanol, i-propanol, n-propanol and ethanol, or without any solvent, at a temperature between 40 and 130 ºC, preferably at 70 to 130 ºC.

Therefore, the temperatures for the enantioselective acylation reaction mentioned in the prior are 55ºC or preferably over 70ºC. These temperatures mean a hurdle for the longevity of the enzymes. The longer the reaction and/or work-up times at high temperatures, the higher the degradation of the enzyme. This kind of enzymes are expensive, and it is industrially demanding that the enzyme could be used several times, i.e., several cycles of separation of (1R,2S)-2,6-dimethyl-2,3-dihydro-1H-inden-1-amine or (1R,2S)-1-amino-2,6-dimethylindane.

Therefore, the present invention provides an alternative to the enzymatic process for the separation of (1R,2S)-2,6-dimethyl-2,3-dihydro-1H-inden-1-amine and it is an object of the present invention to provide a process which solves the problem of the longevity of the enzyme, i.e. the number of cycles of separation of (1R,2S)-2,6-dimethyl-2,3-dihydro-1H-inden-1-amine that an enzyme can be used.

### SUMMARY OF THE INVENTION

A first aspect of the invention is a process for the separation of (1R,2S)-2,6-dimethyl-2,3-dihydro-1H-inden-1-amine with a purity of at least 95% which comprises a first cycle comprising the following steps 1) and 2):
1) reaction of a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent,
2) separation of the amide compound of formula (I) produced in step 1), from the three isomers (1R,1R)-, (1S,2S)- and (15,2R)-2,6-dimethyl-2,3-dihydro-1H-inden-1-amine, 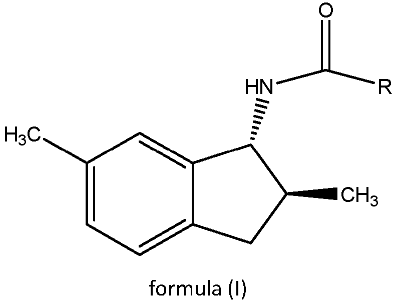
   wherein the process comprises at least one or more cycles of steps 1) and 2), using at least part of the same mass of enzyme *Candida antarctica* as used in the first cycle,
   the process additionally comprising at least one step 3), and at least one step 4), said step 3) is the hydrogenation with a metal catalyst of the mixture of the three isomers (1R,1R)-, (1S,2S)- and (15,2R)-2,6-dimethyl-2,3-dihydro-1H-inden-1-amine obtained in step 2); and said step 4) comprises the reaction of the compound of formula (I) with a base or an acid to produce (1R,2S)-2,6-dimethyl-2,3-dihydro-1H-inden-1-amine.

A second aspect of the invention is a process for the preparation of an amide compound of formula (I) 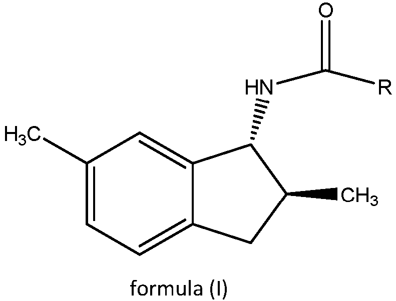 from a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent.

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the invention. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The expression "at least part of the same mass of enzyme *Candida antarctica* as used in the first cycle" in the present invention means that at least part of the enzyme of *Candida antarctica* used in step 1) of the first cycle is used in the second, and optionally more, cycles.

The term "alkyl group" refers to a saturated, linear or branched group, which has between 1 and 4 carbon atoms and is bound to the rest of the molecule by a single bond, including, for example and not restricted to, methyl, ethyl, isopropyl, isobutyl, tert-butyl and similar.

The expressions "the solvent of the reaction in step 1) may comprise", or "the solvent of the reaction may comprise", or "the solvent of the acylating reaction in step 1) may comprise", or "the solvent of the reaction comprises", mean that at least 80% of the solvent in the acylation reaction in step 1) of the present invention is the mentioned solvent.

The expression "purity of at least 95%" for the (1R,2S)-2,6-dimethyl-2,3-dihydro-1H-inden-1-amine isomer means that the enantiomeric and diastereomeric purity of (1R,2S)-2,6-dimethyl-2,3-dihydro-1H-inden-1-amine is 95%, 96%, 97%, 98%, 99% or 100%.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Enzymatic process for the separation of (1R,2S)-2,6-dimethyl-2,3-dihydro-1H-inden-1-amine

The present disclosure relates to a process for the separation of (1R,2S)-2,6-dimethyl-2,3-dihydro-1H-inden-1-amine with a purity of at least 95% which comprises a first cycle comprising the following steps 1) and 2):
1) reaction of a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent,
2) separation of the amide compound of formula (I) produced in step 1), from the three isomers (1R,1R)-, (1S,2S)- and (15,2R)-2,6-dimethyl-2,3-dihydro-1H-inden-1-amine, 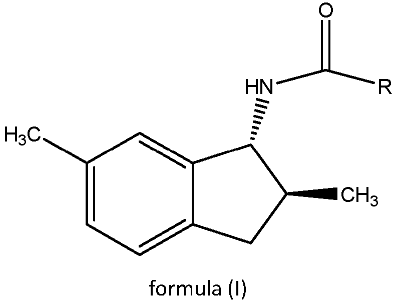
   wherein the process comprises at least one or more cycles of steps 1) and 2), using at least part of the same mass of enzyme *Candida antarctica* as used in the first cycle,
   the process additionally comprising at least one step 3), and at least one step 4), said step 3) is the hydrogenation with a metal catalyst of the mixture of the three isomers (1R,1R)-, (1S,2S)- and (15,2R)-2,6-dimethyl-2,3-dihydro-1H-inden-1-amine obtained in step 2); and said step 4) comprises the reaction of the compound of formula (I) with a base or an acid to produce (1R,2S)-2,6-dimethyl-2,3-dihydro-1H-inden-1-amine.

This process provides an alternative to the existing enzymatic processes for the separation of (1R,2S)-2,6-dimethyl-2,3-dihydro-1H-inden-1-amine from a mixture of the 4 isomers of 2,6-dimethyl-2,3-dihydro-1H-inden-1-amine, and it provides a solution to the problem in the state of the art of the longevity of the enzyme, i.e. the number of cycles of separation of (1R,2S)-2,6-dimethyl-2,3-dihydro-1H-inden-1-amine that an enzyme can be used before the enzyme is damaged and it becomes useless.

The amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine.

The R group of the acylating agent may be -(C₁-C₂-alkyl)-O-(C₁-C₄-alkyl). The R group of the acylating agent may be selected from the group of -CH₂-O-CH₃, -CH₂-O-CH₂-CH₃ or -CH₂-O-CH-(CH₃)₂. The R group of the acylating agent may be -CH₂-O-CH₃.

The R¹ group of the acylating agent may be selected from the group of methyl, ethyl or isopropyl. The R¹ group of the acylating agent may be methyl or isopropyl. The R¹ group of the acylating agent may be isopropyl.

The R group of the acylating agent may be -(C₁-C₂-alkyl)-O-(C₁-C₄-alkyl) and the R¹ group may be selected from the group of methyl, ethyl or isopropyl. The R group of the acylating agent may be selected from the group of -CH₂-O-CH₃, -CH₂-O-CH₂-CH₃ or -CH₂-O-CH-(CH₃)₂ and the R¹ group may be methyl or isopropyl. The R group of the acylating agent may be -CH₂-O-CH₃ and the R¹ group may be isopropyl.

The R group of the acylating agent may be -(C₁-C₂-alkyl)-O-(C₁-C₄-alkyl), the R¹ group may be selected from the group of methyl, ethyl or isopropyl, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The R group of the acylating agent may be -CH₂-O-CH₃, the R¹ group may be isopropyl, and the amount of acylating agent may range from 0.6 to 1.0 equivalents of acylating agent per equivalent of the mixture of four isomers of 2,6-dimethyl-2,3-dihydro-1H-inden-1-amine.

The temperature of the reaction with the acylating agent in step 1) may range from 0ºC to 50ºC, from 10ºC to 45ºC, from 15ºC to 40ºC.

The temperature of the reaction with the acylating agent in step 1) may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The temperature of the reaction with the acylating agent in step 1) may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The temperature of the reaction with the acylating agent in step 1) may range from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The temperature of the reaction with the acylating agent in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The temperature of the reaction with the acylating agent in step 1) may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The temperature of the reaction with the acylating agent in step 1) may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The solvent of the reaction in step 1) may comprise a tertiary alcohol. The solvent of the reaction in step 1) may comprise a tertiary alcohol selected from the group of tert-butyl alcohol or tert-amyl alcohol.

The solvent of the reaction in step 1) may comprise a tertiary alcohol, and the temperature of the reaction in step 1) may range from 0ºC to 50ºC, from 10ºC to 45ºC, from 15ºC to 40ºC. The solvent of the reaction in step 1) may comprise a tertiary alcohol selected from the group of tert-butyl alcohol or tert-amyl alcohol, and the temperature of the reaction in step 1) may range from 10ºC to 45ºC, from 15ºC to 40ºC.

The solvent of the reaction in step 1) may comprise a tertiary alcohol, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The solvent of the reaction in step 1) may comprise a tertiary alcohol selected from the group of tert-butyl alcohol or tert-amyl alcohol, the temperature of the reaction in step 1) may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The solvent of the reaction in step 1) may comprise a tertiary alcohol selected from the group of tert-butyl alcohol or tert-amyl alcohol, the temperature of the reaction in step 1) may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The solvent of the reaction in step 1) may comprise an aromatic hydrocarbon. The solvent of the reaction in step 1) may comprise an aromatic hydrocarbon selected from the group of toluene, cumene, xylenes, cymenes, mesitylene. The solvent of the reaction in step 1) may comprise toluene.

The solvent of the reaction in step 1) may comprise an aromatic hydrocarbon, and the temperature of the reaction in step 1) may range from 0ºC to 50ºC, from 10ºC to 45ºC, from 15ºC to 40ºC. The solvent of the reaction in step 1) may comprise an aromatic hydrocarbon selected from the group of toluene, cumene, xylenes, cymenes, mesitylene, and the temperature of the reaction in step 1) may range from 10ºC to 45ºC, from 15ºC to 40ºC. The solvent of the reaction in step 1) may comprise toluene, and the temperature of the reaction in step 1) may range from 10ºC to 45ºC, from 15ºC to 40ºC.

The solvent of the reaction in step 1) may comprise an aromatic hydrocarbon, the temperature of the reaction in step 1) may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The solvent of the reaction in step 1) may comprise an aromatic hydrocarbon selected from the group of toluene, cumene, xylenes, cymenes, mesitylene, the temperature of the reaction in step 1) may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The solvent of the reaction in step 1) may comprise toluene, the temperature of the reaction in step 1) may range from 10ºC to 45ºC, from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The solvent of the reaction in step 1) may comprise an aromatic hydrocarbon, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The solvent of the reaction in step 1) may comprise an aromatic hydrocarbon selected from the group of toluene, cumene, xylenes, cymenes, mesitylene, the temperature of the reaction in step 1) may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The solvent of the reaction in step 1) may comprise toluene, the temperature of the reaction in step 1) may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

Molecular sieves may be added to the reaction with the acylating agent in step 1) or before the reaction with the acylating agent in step 1), and/or an azeotropic distillation of the an acylating agent and/or of the mixture of the four isomers (1R,1R)-, (1S,2S)-, (1S,2R)- and (1R,2S)-2,6-dimethyl-2,3-dihydro-1H-inden-1-amine may be carried out before the reaction in step 1). The medium pore diameter of the molecular sieves that may be added to the reaction in step 1) may range from 1 Å to 5 Å. The medium pore diameter of the molecular sieves that may be added to the reaction in step 1) may be 4 Å.

The amount of *Candida antarctica* enzyme in the reaction with the acylating agent in step 1) may be between 0.05% and 5%, between 0.5% and 4%, between 1% and 4%, by weight of the amount of mixture of four isomers of 2,6-dimethyl-2,3-dihydro-1H-inden-1-amine.

The amount of *Candida antarctica* enzyme in the reaction in step 1) may be between 0.05% and 5%, between 0.5% and 4%, between 1% and 4%, by weight of the amount of mixture of four isomers of 2,6-dimethyl-2,3-dihydro-1H-inden-1-amine, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The amount of *Candida antarctica* enzyme in the reaction in step 1) may be between 0.5% and 4%, between 1% and 4%, by weight of the amount of mixture of four isomers of 2,6-dimethyl-2,3-dihydro-1H-inden-1-amine, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The amount of *Candida antarctica* enzyme in the reaction in step 1) may be between 1% and 4%, by weight of the amount of mixture of four isomers of 2,6-dimethyl-2,3-dihydro-1H-inden-1-amine, and the amount of acylating agent may range from 0.6 to 1.0 equivalents of acylating agent per equivalent of the mixture of four isomers of 2,6-dimethyl-2,3-dihydro-1H-inden-1-amine.

The amount of *Candida antarctica* enzyme in the reaction in step 1) may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, and the temperature of the reaction may range from 0ºC to 50ºC, from 10ºC to 45ºC, from 15ºC to 40ºC. The amount of *Candida antarctica* enzyme in the reaction in step 1) may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, and the temperature of the reaction may range from 10ºC to 45ºC, from 15ºC to 40ºC. The amount of *Candida antarctica* enzyme in the reaction in step 1) may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, and the temperature of the reaction may range from 15ºC to 40ºC.

The amount of *Candida antarctica* enzyme in the reaction in step 1) may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, and the temperature of the reaction may range from 0ºC to 50ºC, from 10ºC to 45ºC, from 15ºC to 40ºC. The amount of *Candida antarctica* enzyme in the reaction in step 1) may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, and the temperature of the reaction may range from 10ºC to 45ºC, from 15ºC to 40ºC. The amount of *Candida antarctica* enzyme in the reaction in step 1) may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, and the temperature of the reaction may range from 15ºC to 40ºC.

The amount of *Candida antarctica* enzyme in the reaction in step 1) may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, the solvent of the reaction may comprise a tertiary alcohol, and the temperature of the reaction may range from 0ºC to 50ºC, from 10ºC to 45ºC, from 15ºC to 40ºC. The amount of *Candida antarctica* enzyme in the reaction in step 1) may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, the solvent of the reaction may comprise a tertiary alcohol selected from the group of tert-butyl alcohol or tert-amyl alcohol, and the temperature of the reaction may range from 10ºC to 45ºC, from 15ºC to 40ºC. The amount of *Candida antarctica* enzyme in the reaction in step 1) may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, the solvent of the reaction may comprise a tertiary alcohol selected from the group of tert-butyl alcohol or tert-amyl alcohol, and the temperature of the reaction may range from 15ºC to 40ºC.

The amount of *Candida antarctica* enzyme in the reaction in step 1) may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, the solvent of the reaction may comprise an aromatic hydrocarbon, and the temperature of the reaction may range from 0ºC to 50ºC, from 10ºC to 45ºC, from 15ºC to 40ºC. The amount of *Candida antarctica* enzyme in the reaction in step 1) may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, the solvent of the reaction may comprise an aromatic hydrocarbon selected from the group of toluene, cumene, xylenes, cymenes, mesitylene, and the temperature of the reaction may range from 10ºC to 45ºC, from 15ºC to 40ºC. The amount of *Candida antarctica* enzyme in the reaction in step 1) may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, the solvent of the reaction may comprise toluene, and the temperature of the reaction may range from 15ºC to 40ºC.

The volume of solvent in step 1) may be 0 or it may range from 0.5 to 5 volumes of the sum of the volumes of acylating agent and the mixture of the four isomers of 2,6-dimethyl-2,3-dihydro-1H-inden-1-amine. The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, and the solvent of the reaction may comprise a tertiary alcohol. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise a tertiary alcohol selected from the group of tert-butyl alcohol or tert-amyl alcohol.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise toluene.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, and the temperature of the reaction in step 1) may range from 0ºC to 50ºC, from 10ºC to 45ºC, from 15ºC to 40ºC. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, and the temperature of the reaction in step 1) may range from 15ºC to 40ºC.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, and the temperature of the reaction in step 1) may range from 0ºC to 50ºC, from 10ºC to 45ºC, from 15ºC to 40ºC. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, and the temperature of the reaction in step 1) may range from 10ºC to 45ºC, from 15ºC to 40ºC. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise toluene, and the temperature of the reaction in step 1) may range from 10ºC to 45ºC, from 15ºC to 40ºC.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction in step 1) may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction in step 1) may range from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction in step 1) may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction in step 1) may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction in step 1) may range from 10ºC to 45ºC, from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction in step 1) may range from 10ºC to 45ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction in step 1) may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction in step 1) may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction in step 1) may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl), the R¹ group may be selected from the group of methyl, ethyl or isopropyl, and the amount of *Candida antarctica* enzyme may be between 0.05% and 5%, between 0.5% and 4%, between 1% and 4%, by weight of the amount of mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction in step 1) may range from 10ºC to 45ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of *Candida antarctica* enzyme may be between 0.5% and 4%, between 1% and 4%, by weight of the amount of mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction in step 1) may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃, the R¹ group may be isopropyl, and the amount of *Candida antarctica* enzyme may be between 1% and 4%, by weight of the amount of mixture of four isomers of 2,6-dimethyl-2,3-dihydro-1H-inden-1-amine.

The volume of solvent in step 1) may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction in step 1) may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl), the R¹ group may be selected from the group of methyl, ethyl or isopropyl, and the amount of *Candida antarctica* enzyme may be between 0.05% and 5%, between 0.5% and 4%, between 1% and 4%, by weight of the amount of mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction in step 1) may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of *Candida antarctica* enzyme may be between 0.5% and 4%, between 1% and 4%, by weight of the amount of mixture of four isomers of 2,6-dimethyl-2,3-dihydro-1H-inden-1-amine. The volume of solvent in step 1) may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction in step 1) may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃, the R¹ group may be isopropyl, and the amount of *Candida antarctica* enzyme may be between 1% and 4%, by weight of the amount of mixture of four isomers of 2,6-dimethyl-2,3-dihydro-1H-inden-1-amine.

The enzyme in the process of the present invention may be filtered off after step 1) and before step 2).

The unreacted acylating agent after step 1), and the solvent of the reaction of step 1) may be removed before next cycle or before step 3) or before step 4). The removal of the solvent and the unreacted acylating agent may be done by distillation under a pressure that is lower than the atmospheric pressure.

The filtering of the enzyme after step 1) and before step 2) may be carried out before the distillation of the solvent and the unreacted acylating agent. The filtering of the enzyme after step 1) and before step 2) may be carried out before the distillation of the solvent and the unreacted acylating agent under a pressure that may be lower than the atmospheric pressure.

The separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (15,2R)-2,6-dimethyl-2,3-dihydro-1H-inden-1-amine from the reaction mixture after step 1). The separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (1S,2R)-2,6-dimethyl-2,3-dihydro-1H-inden-1-amine from the reaction mixture after step 1) by addition of acid to precipitate the salts of three isomers (1R,1R)-, (1S,2S)- and (1S,2R)-2,6-dimethyl-2,3-dihydro-1H-inden-1-amine.

The enzyme in the process of the present invention may be filtered off after step 1) and before step 2), and the separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (1S,2R)-2,6-dimethyl-2,3-dihydro-1H-inden-1-amine from the reaction mixture after step 1). The enzyme in the process of the present invention may be filtered off after step 1) and before step 2), and the separation in step 2) of the amide compound of formula (I) from the three isomers (1R,1R)-, (1S,2S)- and (1S,2R)-2,6-dimethyl-2,3-dihydro-1H-inden-1-amine may be the separation of the three isomers (1R,1R)-, (1S,2S)- and (15,2R)-2,6-dimethyl-2,3-dihydro-1H-inden-1-amine from the reaction mixture after step 1) by addition of acid to precipitate the salts of three isomers (1R,1R)-, (1S,2S)- and (1S,2R)-2,6-dimethyl-2,3-dihydro-1H-inden-1-amine.

The separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (15,2R)-2,6-dimethyl-2,3-dihydro-1H-inden-1-amine from the reaction mixture after step 1), and the unreacted acylating agent after step 1), and the solvent of the reaction of step 1) may be removed before next cycle or before step 3) or before step 4). The separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (1S,2R)-2,6-dimethyl-2,3-dihydro-1H-inden-1-amine from the reaction mixture after step 1) by addition of acid to precipitate the salts of three isomers (1R,1R)-, (1S,2S)- and (15,2R)-2,6-dimethyl-2,3-dihydro-1H-inden-1-amine, and the unreacted acylating agent after step 1), and the solvent of the reaction of step 1) may be removed before next cycle or before step 3) or before step 4), wherein the removal of the solvent and the unreacted acylating agent may be done by distillation under a pressure that may be lower than the atmospheric pressure.

The enzyme in the process of the present invention may be filtered off after step 1) and before step 2), the separation in step 2) of the amide compound of formula (I) from the three isomers (1R,1R)-, (1S,2S)- and (15,2R)-2,6-dimethyl-2,3-dihydro-1H-inden-1-amine may be the separation of the three isomers (1R,1R)-, (1S,2S)- and (1S,2R)-2,6-dimethyl-2,3-dihydro-1H-inden-1-amine from the reaction mixture after step 1), and the unreacted acylating agent after step 1), and the solvent of the reaction of step 1) may be removed before next cycle or before step 3) or before step 4). The enzyme in the process of the present invention may be filtered off after step 1) and before step 2), the separation in step 2) of the amide compound of formula (I) from the three isomers (1R,1R)-, (1S,2S)- and (1S,2R)-2,6-dimethyl-2,3-dihydro-1H-inden-1-amine may be the separation of the three isomers (1R,1R)-, (1S,2S)- and (1S,2R)-2,6-dimethyl-2,3-dihydro-1H-inden-1-amine from the reaction mixture after step 1) by addition of acid to precipitate the salts of three isomers (1R,1R)-, (1S,2S)- and (1S,2R)-2,6-dimethyl-2,3-dihydro-1H-inden-1-amine, and the unreacted acylating agent after step 1), and the solvent of the reaction of step 1) may be removed before next cycle or before step 3) or before step 4), wherein the removal of the solvent and the unreacted acylating agent may be done by distillation under a pressure that is lower than the atmospheric pressure.

The metal catalyst in the hydrogenation of step 3) may be a catalyst compound with at least one chemical element selected from the groups 8, 9, or 10. The metal catalyst in the hydrogenation of step 3) may be a palladium catalyst. The palladium catalyst may be palladium on activated carbon, or palladium on alumina.

The amount of metal catalyst in the hydrogenation of step 3) may range from 0.5 to 7%, from 0.5 to 5%, from 1 to 5%, by weight of the amount of the mixture of the three isomers (1R,1R)-, (1S,2S)- and (1S,2R)-2,6-dimethyl-2,3-dihydro-1H-inden-1-amine.

The amount of metal catalyst in the hydrogenation of step 3) may range from 0.5 to 7%, from 0.5 to 5%, from 1 to 5%, by weight of the amount of the mixture of the three isomers (1R,1R)-, (1S,2S)- and (1S,2R)-2,6-dimethyl-2,3-dihydro-1H-inden-1-amine, and the metal catalyst may be a catalyst compound with at least one chemical element selected from the groups 8, 9, or 10. The amount of metal catalyst in the hydrogenation of step 3) may range from 0.5 to 7%, from 0.5 to 5%, from 1 to 5%, by weight of the amount of the mixture of the three isomers (1R,1R)-, (1S,2S)- and (1S,2R)-2,6-dimethyl-2,3-dihydro-1H-inden-1-amine, and the metal catalyst may be a palladium catalyst. The amount of metal catalyst in the hydrogenation of step 3) may range from 0.5 to 7%, from 0.5 to 5%, from 1 to 5%, by weight of the amount of the mixture of the three isomers (1R,1R)-, (1S,2S)- and (1S,2R)-2,6-dimethyl-2,3-dihydro-1H-inden-1-amine, and the metal catalyst may be palladium on activated carbon, or palladium on alumina.

The step 1) in the process of the invention is carried out under a protective gas atmosphere.

### Enzymatic process for the preparation of a carbamate compound of (1R,2S)-2,6-dimethyl-2,3-dihydro-1H-inden-1-amine

The present invention also relates to a process for the preparation of a carbamate compound of formula (I) 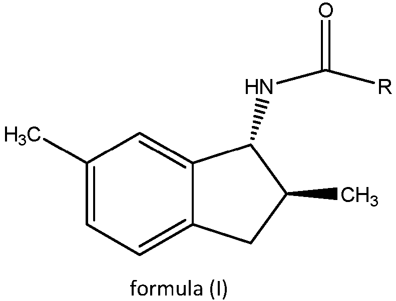 from a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent.

The amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine.

The R group of the acylating agent may be -(C₁-C₂-alkyl)-O-(C₁-C₄-alkyl). The R group of the acylating agent may be selected from the group of -CH₂-O-CH₃, -CH₂-O-CH₂-CH₃ or -CH₂-O-CH-(CH₃)₂. The R group of the acylating agent may be -CH₂-O-CH₃.

The R¹ group of the acylating agent may be selected from the group of methyl, ethyl or isopropyl. The R¹ group of the acylating agent may be methyl or isopropyl. The R¹ group of the acylating agent may be isopropyl.

The R group of the acylating agent may be -(C₁-C₂-alkyl)-O-(C₁-C₄-alkyl) and the R¹ group may be selected from the group of methyl, ethyl or isopropyl. The R group of the acylating agent may be selected from the group of -CH₂-O-CH₃, -CH₂-O-CH₂-CH₃ or -CH₂-O-CH-(CH₃)₂ and the R¹ group may be methyl or isopropyl. The R group of the acylating agent may be -CH₂-O-CH₃ and the R¹ group may be isopropyl.

The R group of the acylating agent may be -(C₁-C₂-alkyl)-O-(C₁-C₄-alkyl), the R¹ group may be selected from the group of methyl, ethyl or isopropyl, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The R group of the acylating agent may be -CH₂-O-CH₃, the R¹ group may be isopropyl, and the amount of acylating agent may range from 0.6 to 1.0 equivalents of acylating agent per equivalent of the mixture of four isomers of 2,6-dimethyl-2,3-dihydro-1H-inden-1-amine.

The temperature of the reaction with the acylating agent may range from 0ºC to 50ºC, from 10ºC to 45ºC, from 15ºC to 40ºC.

The temperature of the reaction with the acylating agent may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The temperature of the reaction with the acylating agent may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The temperature of the reaction with the acylating agent may range from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The temperature of the reaction with the acylating agent may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The temperature of the reaction with the acylating agent may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The temperature of the reaction with the acylating agent may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The solvent of the reaction may comprise a tertiary alcohol. The solvent of the reaction may comprise a tertiary alcohol selected from the group of tert-butyl alcohol or tert-amyl alcohol.

The solvent of the reaction may comprise a tertiary alcohol, and the temperature of the reaction may range from 0ºC to 50ºC, from 10ºC to 45ºC, from 15ºC to 40ºC. The solvent of the reaction may comprise a tertiary alcohol selected from the group of tert-butyl alcohol or tert-amyl alcohol, and the temperature of the reaction may range from 10ºC to 45ºC, from 15ºC to 40ºC.

The solvent of the reaction may comprise a tertiary alcohol, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The solvent of the reaction may comprise a tertiary alcohol selected from the group of tert-butyl alcohol or tert-amyl alcohol, the temperature of the reaction may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The solvent of the reaction may comprise a tertiary alcohol selected from the group of tert-butyl alcohol or tert-amyl alcohol, the temperature of the reaction may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The solvent of the reaction may comprise an aromatic hydrocarbon. The solvent of the reaction may comprise an aromatic hydrocarbon selected from the group of toluene, cumene, xylenes, cymenes, mesitylene. The solvent of the reaction may comprise toluene.

The solvent of the reaction may comprise an aromatic hydrocarbon, and the temperature of the reaction may range from 0ºC to 50ºC, from 10ºC to 45ºC, from 15ºC to 40ºC. The solvent of the reaction may comprise an aromatic hydrocarbon selected from the group of toluene, cumene, xylenes, cymenes, mesitylene, and the temperature of the reaction may range from 10ºC to 45ºC, from 15ºC to 40ºC. The solvent of the reaction may comprise toluene, and the temperature of the reaction may range from 10ºC to 45ºC, from 15ºC to 40ºC.

The solvent of the reaction may comprise an aromatic hydrocarbon, the temperature of the reaction may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The solvent of the reaction may comprise an aromatic hydrocarbon selected from the group of toluene, cumene, xylenes, cymenes, mesitylene, the temperature of the reaction may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The solvent of the reaction may comprise toluene, the temperature of the reaction may range from 10ºC to 45ºC, from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The solvent of the reaction may comprise an aromatic hydrocarbon, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The solvent of the reaction may comprise an aromatic hydrocarbon selected from the group of toluene, cumene, xylenes, cymenes, mesitylene, the temperature of the reaction may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The solvent of the reaction may comprise toluene, the temperature of the reaction may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

Molecular sieves may be added to the reaction with the acylating agent or before the reaction with the acylating agent, and/or an azeotropic distillation of the an acylating agent and/or of the mixture of the four isomers (1R,1R)-, (1S,2S)-, (1S,2R)- and (1R,2S)-2,6-dimethyl-2,3-dihydro-1H-inden-1-amine may be carried out before the reaction. The medium pore diameter of the molecular sieves that may be added to the reaction may range from 1 Å to 5 Å. The medium pore diameter of the molecular sieves that may be added to the reaction may be 4 Å.

The amount of *Candida antarctica* enzyme in the reaction with the acylating agent may be between 0.05% and 5%, between 0.5% and 4%, between 1% and 4%, by weight of the amount of mixture of four isomers of 2,6-dimethyl-2,3-dihydro-1H-inden-1-amine.

The amount of *Candida antarctica* enzyme in the reaction may be between 0.05% and 5%, between 0.5% and 4%, between 1% and 4%, by weight of the amount of mixture of four isomers of 2,6-dimethyl-2,3-dihydro-1H-inden-1-amine, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The amount of *Candida antarctica* enzyme in the reaction may be between 0.5% and 4%, between 1% and 4%, by weight of the amount of mixture of four isomers of 2,6-dimethyl-2,3-dihydro-1H-inden-1-amine, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The amount of *Candida antarctica* enzyme in the reaction may be between 1% and 4%, by weight of the amount of mixture of four isomers of 2,6-dimethyl-2,3-dihydro-1H-inden-1-amine, and the amount of acylating agent may range from 0.6 to 1.0 equivalents of acylating agent per equivalent of the mixture of four isomers of 2,6-dimethyl-2,3-dihydro-1H-inden-1-amine.

The amount of *Candida antarctica* enzyme in the reaction may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, and the temperature of the reaction may range from 0ºC to 50ºC, from 10ºC to 45ºC, from 15ºC to 40ºC. The amount of *Candida antarctica* enzyme in the reaction may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, and the temperature of the reaction may range from 10ºC to 45ºC, from 15ºC to 40ºC. The amount of *Candida antarctica* enzyme in the reaction may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, and the temperature of the reaction may range from 15ºC to 40ºC.

The amount of *Candida antarctica* enzyme in the reaction may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, and the temperature of the reaction may range from 0ºC to 50ºC, from 10ºC to 45ºC, from 15ºC to 40ºC. The amount of *Candida antarctica* enzyme in the reaction may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, and the temperature of the reaction may range from 10ºC to 45ºC, from 15ºC to 40ºC. The amount of *Candida antarctica* enzyme in the reaction may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, and the temperature of the reaction may range from 15ºC to 40ºC.

The amount of *Candida antarctica* enzyme in the reaction may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, the solvent of the reaction may comprise a tertiary alcohol, and the temperature of the reaction may range from 0ºC to 50ºC, from 10ºC to 45ºC, from 15ºC to 40ºC. The amount of *Candida antarctica* enzyme in the reaction may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, the solvent of the reaction may comprise a tertiary alcohol selected from the group of tert-butyl alcohol or tert-amyl alcohol, and the temperature of the reaction may range from 10ºC to 45ºC, from 15ºC to 40ºC. The amount of *Candida antarctica* enzyme in the reaction may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, the solvent of the reaction may comprise a tertiary alcohol selected from the group of tert-butyl alcohol or tert-amyl alcohol, and the temperature of the reaction may range from 15ºC to 40ºC.

The amount of *Candida antarctica* enzyme in the reaction may be between 0.05% and 5%, between 0.5% and 4%, between 1% and 4%, by weight of the amount of mixture of four isomers of 2,6-dimethyl-2,3-dihydro-1H-inden-1-amine, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl, the solvent of the reaction may comprise an aromatic hydrocarbon, and the temperature of the reaction may range from 0ºC to 50ºC, from 10ºC to 45ºC, from 15ºC to 40ºC. The amount of *Candida antarctica* enzyme in the reaction may be between 0.5% and 4%, between 1% and 4%, by weight of the amount of mixture of four isomers of 2,6-dimethyl-2,3-dihydro-1H-inden-1-amine, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl, the solvent of the reaction may comprise an aromatic hydrocarbon selected from the group of toluene, cumene, xylenes, cymenes, mesitylene, and the temperature of the reaction may range from 10ºC to 45ºC, from 15ºC to 40ºC. The amount of *Candida antarctica* enzyme in the reaction may be between 1% and 4%, by weight of the amount of mixture of four isomers of 2,6-dimethyl-2,3-dihydro-1H-inden-1-amine, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl, the solvent of the reaction may comprise toluene, and the temperature of the reaction may range from 15ºC to 40ºC.

The volume of solvent may be 0 or it may range from 0.5 to 5 volumes of the sum of the volumes of acylating agent and the mixture of the four isomers of 2,6-dimethyl-2,3-dihydro-1H-inden-1-amine. The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, and the solvent of the reaction may comprise a tertiary alcohol. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise a tertiary alcohol selected from the group of tert-butyl alcohol or tert-amyl alcohol.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise toluene.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, and the temperature of the reaction may range from 0ºC to 50ºC, from 10ºC to 45ºC, from 15ºC to 40ºC. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, and the temperature of the reaction may range from 15ºC to 40ºC.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, and the temperature of the reaction may range from 0ºC to 50ºC, from 10ºC to 45ºC, from 15ºC to 40ºC. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, and the temperature of the reaction may range from 10ºC to 45ºC, from 15ºC to 40ºC. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise toluene, and the temperature of the reaction may range from 10ºC to 45ºC, from 15ºC to 40ºC.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction may range from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction may range from 0ºC to 50ºC, from 10ºC to 45ºC, from 15ºC to 40ºC, and the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction may range from 10ºC to 45ºC, from 15ºC to 40ºC, and the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction may range from 10ºC to 45ºC, from 15ºC to 40ºC, and the amount of acylating agent may range from 0.6 to 1.0 equivalents of acylating agent per equivalent of the mixture of four isomers of 2,6-dimethyl-2,3-dihydro-1H-inden-1-amine.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction may range from 10ºC to 45ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl) and the R¹ group may be selected from the group of methyl, ethyl or isopropyl. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂ and the R¹ group may be methyl or isopropyl. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃ and the R¹ group may be isopropyl.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl), the R¹ group may be selected from the group of methyl, ethyl or isopropyl, and the amount of *Candida antarctica* enzyme may be between 0.05% and 5%, between 0.5% and 4%, between 1% and 4%, by weight of the amount of mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction may range from 10ºC to 45ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of *Candida antarctica* enzyme may be between 0.5% and 4%, between 1% and 4%, by weight of the amount of mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, the solvent of the reaction may comprise a tertiary alcohol selected from the group of tert-butyl alcohol or tert-amyl alcohol, the temperature of the reaction may range from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃, the R¹ group may be isopropyl, and the amount of *Candida antarctica* enzyme may be between 1% and 4%, by weight of the amount of mixture of four isomers of 2,6-dimethyl-2,3-dihydro-1H-inden-1-amine.

The volume of solvent may range from 0.5 to 5 volumes, from 1 to 4 volumes of the sum of the volumes of acylating agent and the mixture of the four isomers of 2,6-dimethyl-2,3-dihydro-1H-inden-1-amine, the solvent of the reaction may comprise an aromatic hydrocarbon, the temperature of the reaction may range from 0ºC to 50ºC, from 10ºC to 45ºC, from 15ºC to 40ºC, the amount of acylating agent may range from 0.4 to 2.5, from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be -(C₁-C₂-alkyl)-O-(C₁-C₄-alkyl), the R¹ group may be selected from the group of methyl, ethyl or isopropyl, and the amount of *Candida antarctica* enzyme may be between 0.05% and 5%, between 0.5% and 4%, between 1% and 4%, by weight of the amount of mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise an aromatic hydrocarbon selected from the group of toluene, cumene, xylenes, cymenes, mesitylene, the temperature of the reaction may range from 10ºC to 45ºC, from 15ºC to 40ºC, the amount of acylating agent may range from 0.4 to 1.6, from 0.6 to 1.0 equivalents of acylating agent per equivalent of the mixture of four isomers of 2,6-dimethyl-2,3-dihydro-1H-inden-1-amine, the R group of the acylating agent may be selected from the group of -CH₂-O-CH₃, -CH₂-O-CH₂-CH₃ or -CH₂-O-CH-(CH₃)₂, the R¹ group may be methyl or isopropyl, and the amount of *Candida antarctica* enzyme may be between 0.5% and 4%, between 1% and 4%, by weight of the amount of mixture of four isomers of 2,6-dimethyl-2,3-dihydro-1H-inden-1-amine. The volume of solvent may range from 1 to 4 volumes of the sum of the volumes of acylating agent and the mixture of the four isomers of 2,6-dimethyl-2,3-dihydro-1H-inden-1-amine, and the solvent of the reaction may comprise toluene, the temperature of the reaction may range from 10ºC to 45ºC, from 15ºC to 40ºC, the amount of acylating agent may range from 0.6 to 1.0 equivalents of acylating agent per equivalent of the mixture of four isomers of 2,6-dimethyl-2,3-dihydro-1H-inden-1-amine, the R group of the acylating agent may be -CH₂-O-CH₃, the R¹ group may be isopropyl, and the amount of *Candida antarctica* enzyme may be between 1% and 4%, by weight of the amount of mixture of four isomers of 2,6-dimethyl-2,3-dihydro-1H-inden-1-amine.

The process for the preparation of the carbamate compound of formula (I) is carried out under a protective gas atmosphere.

The following examples are included for illustrative purposes only and should not be construed as limitations on the invention claimed herein.

### Examples

### Example 1: Acylation reaction of a mixture of 4 isomers of 2,6-dimethyl-2,3-dihydro-1H-inden-1-amine with a tertiary alcohol as solvent

45 gr of a mixture of the 4 isomers of 2,6-dimethyl-2,3-dihydro-1H-inden-1-amine containing 40:40:10:10 of the isomers (1R,2S):(1S,2R):(1R,2R):(1S,2S) is dissolved in 22.5 ml of tert-butanol. Isopropyl 2-methoxyacetate (29.5 gr, 0.8 equivalents) is added at once to the solution. The enzyme *Candida antarctica* of commercial name IMMCALBY-ADS-11 (900 mg, 0.02 equivalents) and molecular sieves with a medium pore diameter of 4 Å (8 gr) are added at 25ºC to the solution of 2,6-dimethyl-2,3-dihydro-1H-inden-1-amine and the acylating agent. The reaction is stirred for 22 hours at 25ºC.

At the end of the reaction at 22 hours, the enzyme is filtered off and recovered, and it is used again in a second reaction with new 2,6-dimethyl-2,3-dihydro-1H-inden-1-amine, acylating agent and solvent in the same quantities and conditions as in the first reaction.

The process of reaction and filtration is repeated for a third and fourth reactions. The conversion rate from (1R,2S)-2,6-dimethyl-2,3-dihydro-1H-inden-1-amine to N-((1R,2S)-2,6-dimethyl-2,3-dihydro-1H-inden-1-yl)-2-methoxyacetamide (M233) for the four reactions is shown in the following Table 1:

**Table 1**

| Acylation reaction | Conversion rate to M233 after 22 h of reaction at 25ºC |
|---|---|
| 1^{st} | 99.2% |
| 2^{nd} | 92.2% |
| 3^{rd} | 85.6% |
| 4^{th} | 80.2% |

It is observed that when the solvent of the acylation reaction is a tertiary alcohol, the enzyme *Candida antarctica* is not damaged, and it can be used for several times, at least 4 times. When the same enzyme is used several times, the conversion rate to N-((1R,2S)-2,6-dimethyl-2,3-dihydro-1H-inden-1-yl)-2-methoxyacetamide is maintained for at least 4 reactions, and the conversion is a high conversion.

### Example 2: Comparative Example for the acylation reaction of a mixture of 4 isomers of 2,6-dimethyl-2,3-dihydro-1H-inden-1-amine with a primary alcohol.

The acylation reaction of Example 1 is repeated, but it starts from 10 gr of mixture of 2,6-dimethyl-2,3-dihydro-1H-inden-1-amine, 20 ml of methanol, 8.2 gr of isopropyl 2-methoxyacetate as acylating agent, and 0.2 gr of IMMCALBY-ADS-11. The reaction is stirred for 18 hours at 25ºC, and the conversion rate from (1R,2S)-2,6-dimethyl-2,3-dihydro-1H-inden-1-amine to N-((1R,2S)-2,6-dimethyl-2,3-dihydro-1H-inden-1-yl)-2-methoxyacetamide is only 4%. The acylation reaction is not repeated due to the low conversion rate.

### Example 3: Comparative Example for the acylation reaction of a mixture of 4 isomers of 2,6-dimethyl-2,3-dihydro-1H-inden-1-amine with a secondary alcohol

The acylation reaction of Example 1 is repeated, but starting from 10 gr of mixture of 2,6-dimethyl-2,3-dihydro-1H-inden-1-amine, 20 ml of isopropanol, 8.2 gr of isopropyl 2-methoxyacetate as acylating agent, and 0.2 gr of IMMCALBY-ADS-11. The reaction is stirred for 18 hours at 25ºC, and the conversion rate from (1R,2S)-2,6-dimethyl-2,3-dihydro-1H-inden-1-amine to N-((1R,2S)-2,6-dimethyl-2,3-dihydro-1H-inden-1-yl)-2-methoxyacetamide is only 64%. The acylation reaction is not repeated due to the low conversion rate.

## Claims

1. A process for the separation of (1R,2S)-2,6-dimethyl-2,3-dihydro-1H-inden-1-amine with a purity of at least 95% which comprises a first cycle comprising the following steps 1) and 2):
1) reaction of a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent,
2) separation of the amide compound of formula (I) produced in step 1), from the three isomers (1R,1R)-, (1S,2S)- and (15,2R)-2,6-dimethyl-2,3-dihydro-1H-inden-1-amine, 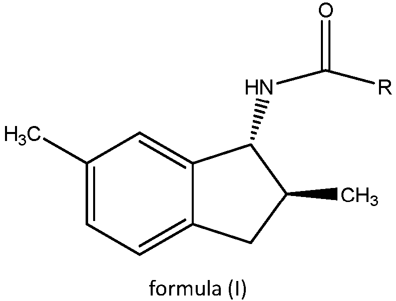
wherein the process comprises at least one or more cycles of steps 1) and 2), using at least part of the same mass of enzyme *Candida antarctica* as used in the first cycle,
the process additionally comprising at least one step 3), and at least one step 4), said step 3) is the hydrogenation with a metal catalyst of the mixture of the three isomers (1R,1R)-, (1S,2S)- and (15,2R)-2,6-dimethyl-2,3-dihydro-1H-inden-1-amine obtained in step 2); and said step 4) comprises the reaction of the compound of formula (I) with a base or an acid to produce (1R,2S)-2,6-dimethyl-2,3-dihydro-1H-inden-1-amine.

2. The process according to claim 1, wherein the amount of acylating agent ranges from 0.4 to 2.5 equivalents of acylating agent per equivalent of the mixture of four isomers of 2,6-dimethyl-2,3-dihydro-1H-inden-1-amine.

3. The process according to any of previous claims, wherein the R group of the acylating agent is -(C₁-C₂-alkyl)-O-(C₁-C₄-alkyl).

4. The process according to any of claims 1 or 2, wherein the R¹ group of the acylating agent is selected from the group of methyl, ethyl or isopropyl.

5. The process according to any of previous claims, wherein the temperature of the reaction with the acylating agent in step 1) ranges from 0ºC to 50ºC.

6. The process according to any of previous claims, wherein the tertiary alcohol of the reaction with the acylating agent in step 1) is selected from the group of tert-butyl alcohol or tert-amyl alcohol.

7. The process according to any of claims 1 to 5, wherein the aromatic hydrocarbon of the reaction with the acylating agent in step 1) is selected from the group of toluene, cumene, xylenes, cymenes, mesitylene.

8. The process according to any of previous claims, wherein the amount of *Candida antarctica* enzyme in the reaction with the acylating agent in step 1) is between 0.05% and 5% by weight of the amount of mixture of four isomers of 2,6-dimethyl-2,3-dihydro-1H-inden-1-amine.

9. The process according to any of previous claims, wherein the volume of solvent in step 1) is 0 or it ranges from 0.5 to 5 volumes of the sum of the volumes of acylating agent and the mixture of the four isomers of 2,6-dimethyl-2,3-dihydro-1H-inden-1-amine.

10. The process according to any of previous claims, wherein the enzyme is filtered off after step 1) and before step 2).

11. The process according to any of previous claims, wherein the unreacted acylating agent after step 1), and the solvent of the reaction of step 1) are removed before next cycle or before step 3) or step 4).

12. The process according to any of previous claims, wherein the separation of the step 2) is the separation of the three isomers (1R,1R)-, (1S,2S)- and (1S,2R)-2,6-dimethyl-2,3-dihydro-1H-inden-1-amine from the reaction mixture after step 1).

13. The process according to any of previous claims, wherein the metal catalyst in the hydrogenation of step 3) is a catalyst compound with at least one chemical element selected from the groups 8, 9, or 10.

14. The process according to any of any of previous claims, wherein the amount of metal catalyst ranges from 0.5 to 7% by weight of the amount of the mixture of the three isomers (1R,1R)-, (1S,2S)- and (15,2R)-2,6-dimethyl-2,3-dihydro-1H-inden-1-amine.

15. A process for the preparation of a carbamate compound of formula (I) 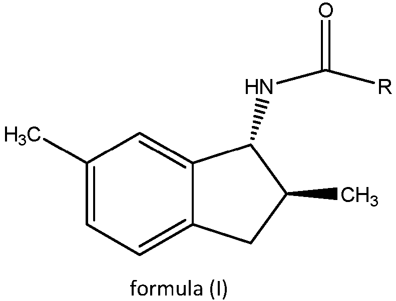 from a mixture of the four isomers (1R,1R)-, (1S,2S)-, (1S,2R)- and (1R,2S)-2,6-dimethyl-2,3-dihydro-1H-inden-1-amine with an acylating agent of formula R-C(=O)OR¹, wherein R is selected from the group of -(C₁-C₂-alkyl)-O-(C₁-C₄-alkyl) or -(C₁-C₄-alkyl), and R¹ is a group -(C₁-C₄-alkyl), in the presence of an enzyme which is *Candida antarctica,* wherein the solvent of the reaction comprises a tertiary alcohol, or an aromatic hydrocarbon, or mixtures thereof, or there is no solvent.
